# EUROPÄISCHE PATENTSCHRIFT

(11) **EP 1 919 522 B1**
(45) Veröffentlichungstag und Bekanntmachung des Hinweises auf die Patenterteilung: **04.07.2012**
(21) Anmeldenummer: 06777857.1
(22) Anmeldetag: 20.07.2006
(51) Int. Cl.: A23L 3/32, C02F 1/50, A61L 2/23, A61L 2/232, A61L 2/238, A61L 2/03

(54) **STERILISATIONSSYSTEM ZUM STERILISIEREN UND/ODER INAKTIVIEREN DER AKTIVITÄT VON MIKROORGANISMEN IN FLÜSSIGKEITEN UND GASEN SOWIE VERFAHREN ZUM STERILISIEREN UND/ODER INAKTIVIEREN**
STERILISATION SYSTEM FOR STERILISING AND/OR NEUTRALISING THE ACTIVITY OF MICRO-ORGANISMS IN LIQUIDS AND GASSES, AND STERILISATION AND/OR NEUTRALISATION PROCESS
SYSTEME DE STERILISATION CONCU POUR STERILISER ET/OU INACTIVER L'ACTIVITE DE MICRO-ORGANISMES DANS DES LIQUIDES ET DES GAZ ET PROCEDES POUR STERILISER ET/OU INACTIVER

(30) Priorität: 05.08.2005 DE 102005037849
(43) Veröffentlichungstag der Anmeldung: 14.05.2008
(73) Patentinhaber: SIEMENS AKTIENGESELLSCHAFT, 80333 München (DE)
(72) Erfinder: JENSEN, Jens, Dahl, 14050 Berlin (DE); RÖNSCH, Hendrik, 10711 Berlin (DE)
(74) Vertreter: Wolff, Harry
(86) Internationale Anmeldenummer: PCT/EP2006/064448
(87) Internationale Veröffentlichungsnummer: WO 2007/017354

(56) Entgegenhaltungen:
- WO-A-98/27896
- WO-A-2004/045577
- DE-A1- 19 800 294
- FR-A- 2 240 020
- GB-A- 202 143

## Beschreibung

Das Gebiet der Erfindung betrifft die Sterilisation / Inaktivierung der Aktivität von Mikroorganismen in Flüssigkeiten und Gasen. Dies wird üblicherweise durch Techniken wie
- Versetzen der Flüssigkeit /des Gases mit chemischen Agentien wie etwa Chlor, Ozon (insbesondere bei Trinkwasser) oder auch Metallionen wie Kupfer- oder Silberverbindungen
- Radioaktive oder UV-Strahlung
- kurzzeitige Erhitzung (Pasteurisierung)
durchgeführt. Diese Techniken haben jedoch den Nachteil, dass oft auch die Flüssigkeit oder das Gas, welches sterilisiert werden soll, mit beeinflusst wird, etwa dadurch, dass die chemischen Sterilisationsmittel in der Flüssigkeit verbleiben. Auch bei Erhitzen oder Versetzen mit Strahlung lässt sich eine Beeinflussung nicht ausschließen, etwa bei Lebensmitteln.

Verschiedene Sterilisationssysteme sind aus dem Stand der Technik bereits bekannt. Beispielsweise wird in der WO 98/27896 A vorgeschlagen, dass optische Linsen aus einem antimikrobiellen Polymer gefertigt werden können. Dieses enthält Partikel mit antimikrobiellen Eigenschaften. Gemäß der GB 202,143 wird vorgeschlagen, dass Kieselguhr sowohl mit Silber als auch mit Platin beschichtet werden kann, so dass sich zwischen den beiden Metallen ein antimicrobiell wirkendes Potential ausbildet. Modifizierte Kieselguhr kann beispielsweise in einem Filter für bakterienkontaminiertes Wasser eingesetzt werden. Der antimikrobielle Effekt aufgrund der Ausbildung elektrischer Potentiale auf Oberflächen befindlicher Metallpaarungen ist auch aus der FR 2 240 020 A und der WO2004/045577 A1 bekannt.

Es stellt sich daher die Aufgabe, ein Sterilisationssystem zu schaffen, welches in der Lage ist, weitgehend beeinflussungsfrei ein Sterilisation und/oder Inaktivierung der Aktivität von Mikroorganismen in Flüssigkeiten und Gasen durchzuführen.

Diese Aufgabe wird durch ein Sterilisationssystem gemäß den Merkmalen des Anspruchs 1 gelöst. Demgemäß wird ein Sterilisationssystem für die Sterilisation und/oder Inaktivierung der Aktivität von Mikroorganismen in Flüssigkeiten und/oder Gasen bereitgestellt, wobei die Sterilisation dadurch bewirkt wird, dass die Mikroorganismen einer Potentialdifferenz von ≥200 bis ≤1000 mV ausgesetzt werden. Erfindungsgemäß enthalten die Partikel zusätzlich mindestens eine ferromagnetische Komponente. Dadurch ist es möglich, die Partikel z.B. in einem Flussreaktor durch Anlegen eines geeigneten Magnetfeldes zu fixieren.

Magnetische Nanopartikel sowie ein Verfahren zur Herstellung dieser Partikel sind beispielsweise in der US 2003 0190475 aufgeführt; diese Nanopartikel sind jedoch für ein Sterilisationssystem gemäß der vorliegenden Erfindung nicht geeignet.

Gemäß einer bevorzugten Ausführungsform der Erfindung in den Partikel die ferromagnetische Komponente als Kern vorgesehen ist, welcher von dem Oxidationsmittel und/oder dem Reduktionsmittel umgeben ist. Dies hat sich als besonders günstige Anordnung herausgestellt.

Gemäß einer bevorzugten Ausführungsform der Erfindung ist die ferromagnetische Komponente ausgewählt aus der Gruppe Fe, Ni, Co oder Mischungen davon. Diese Materialien haben sich als besonders vorteilhaft in der Praxis erwiesen.

Unter Sterilisation im Sinne der vorliegenden Erfindung wird insbesondere eine Desinfektion und/oder Sterilisation verstanden. Desinfektion bedeutet insbesondere eine Keimreduktion um einen Faktor von mindestens 10⁻⁵, d.h. von ursprünglich 100.000 vermehrungsfähigen Keimen (so genannte koloniebildende Einheiten - KBE) überlebt nicht mehr als ein einzelner Keim. Bevorzugt wird unter Sterilisation im Sinne der vorliegenden Erfindung eine Inaktivierung der Flüssigkeit und/oder des Gases verstanden, wobei der Term "Inaktivierung" insbesondere im Sinne einer Reduktion keimbildender Einheiten um einen Faktor von höchstens 10⁻⁶ verstanden wird, d.h. von einer Million Keimen überlebt maximal ein Keim.

Unter Mikroorganismen im Sinne der vorliegenden Erfindung werden insbesondere Bakterien, Pilze, tierische sowie pflanzliche Ein- und Mehrzeller, aber auch DNA (und Fragmente), RNA (und Fragmente), Plasmide und ähnliche Artefakte sowie Viren und Sporen verstanden.

Unter Potentialdifferenz im Sinne der vorliegenden Erfindung wird insbesondere die Differenz zwischen elektrochemisch messbaren Korrosionspotentialen in jedem Typ von Elektrolyt verstanden. Im Sinne der vorliegenden Erfindung werden Korrosionspotentiale insbesondere als Standardwerte gegenüber eine Standard-Referenzelektrode, z. B. Wasserstoffelektrode gemessen, wodurch die einzelnen metallischen Elemente sich in eine geordnete Spannungsreihe einordnen lassen. Insbesondere hier bevorzugt ist die Standardspannungsreihe, gemessen in Wasser bei 25 Grad Celsius gegenüber eine Standard-Wasserstoff-Referenzelektrode.

Ein Sterilisationssystem gemäß der vorliegenden Erfindung zeichnet sich unter anderem durch einen oder mehrere der folgenden Vorteile aus:
- Dadurch, dass kaum Materialien verbraucht werden, ist es sehr ressourcenschonend und zudem meist wartungsarm.
- Das zu sterilisierende Fluid wird bei den meisten Anwendungen nicht oder so gut wie gar nicht beeinflusst, so dass sich das erfindungsgemäße Sterilisationssystem auch für die Sterilisation von Lebensmitteln eignet.
- Dadurch, dass die Sterilisation durch Ausnutzung einer Potentialdifferenz erfolgt, ist der Sterilisationsvorgang gegenüber den meisten anderen Verfahren aus dem Stand der Technik zudem sehr effizient und zeitsparend.

Bevorzugt werden die Mikroorganismen einer Potentialdifferenz von ≥300 bis ≤900 mV ausgesetzt, noch mehr bevorzugt ≥400 bis ≤800 mV sowie am meisten bevorzugt ≥500 bis ≤700 mV.

Gemäß einer bevorzugten Ausführungsform der Erfindung umfasst das System Partikel, bei denen mindestens ein Oxidationsmittel und mindestens ein Reduktionsmittel so angeordnet sind, dass eine Potentialdifferenz von ≥200 bis ≤1000 mV erreicht wird. Dadurch ist es möglich, eine große Menge an Fluid bei hohem Durchsatz zu sterilisieren. Bevorzugt umfasst das System Partikel, bei denen mindestens ein Oxidationsmittel und mindestens ein Reduktionsmittel so angeordnet sind, dass eine Potentialdifferenz von ≥300 bis ≤900 mV ausgesetzt, noch mehr bevorzugt ≥400 bis ≤800 mV sowie am meisten bevorzugt ≥500 bis ≤700 mV erreicht wird.

Es sei darauf hingewiesen, dass im Sinne der elektrochemischen Nomenklatur das Oxidationsmittel als Kathode und das Reduktionsmittel als Anode wirkt bzw. bezeichnet werden kann.

Gemäß einer bevorzugten Ausführungsform der Erfindung weisen die Partikel einen Partikeldurchmesser von ≥20 nm bis ≤10 µm auf. Partikel dieser Größe sind für eine Sterilisation besonders geeignet, da hier zum einen das Oberfläche/VolumenVerhältnis besonders günstig ist, auf der anderen Seite die Partikel nicht so klein sind, dass sie nur noch schlecht handhabbar sind. Bevorzugt weisen die Partikel einen Partikeldurchmesser von ≥50 nm bis ≤1 µm, noch bevorzugt ≥100 nm bis ≤800 nm, sowie am meisten bevorzugt von 200 nm bis ≤500 nm auf.

Gemäß einer bevorzugten Ausführungsform der Erfindung weist das Oxidationsmittel und/oder das Reduktionsmittel eine Austauschstromdichte von ≥10⁻³ A/cm² bis ≤100 A/cm² auf. Durch eine solche Austauschstromdichte wird eine gleichbleibend günstige Potentialdifferenz auch bei längerem Einsatz der Partikel aufrechterhalten. Bevorzugt weist das Oxidationsmittel und/oder das Reduktionsmittel eine Austauschstromdichte von ≥ 10⁻² A/cm² bis ≤ 20 A/cm², noch bevorzugter ≥ 0,1 A/cm² bis ≤ 15 A/cm², sowie am meisten bevorzugt ≥ 0,15 A/cm² bis ≤ 10 A/cm² auf.

Gemäß einer bevorzugten Ausführungsform der Erfindung ist das Oxidationsmittel ausgewählt aus der Gruppe Ag, Au, Cu, Co, Ni oder Mischungen davon. Diese Oxidationsmittel haben sich in der Praxis als die besten geeigneten Oxidationsmittel für die vorliegende Erfindung gezeigt.

Gemäß einer bevorzugten Ausführungsform der Erfindung ist das Reduktionsmittel ausgewählt aus der Gruppe Pd, Pt, Rh, Ir oder Mischungen davon. Diese Reduktionsmittel haben sich in der Praxis als die besten geeigneten Reduktionsmittel für die vorliegende Erfindung gezeigt.

Die vorliegende Erfindung bezieht sich außerdem auf ein Verfahren zum Sterilisieren von Flüssigkeiten oder Gasen, enthaltend die Schritte:
- Bereitstellen eines wie oben beschriebenen Sterilisationssystems in einem Reaktor
- Fixieren des Sterilisationssystems durch Anlegen eines magnetischen Feldes
- Durchleiten der zu sterilisierenden Flüssigkeit oder des Gases durch den Reaktor

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Zeichnungen, in denen - beispielhaft - mehrere Ausführungsbeispiele des erfindungsgemäßen Sterilisationssystems dargestellt sind. In den Zeichnungen zeigt:
- Fig.1: ein Sterilisationssystem in Form von Partikeln, angeordnet in einem Flussreaktor gemäß einer ersten Ausführungsform der Erfindung in - sehr schematischer - Schnittansicht; sowie
- Fig.2: ein Partikel aus Fig.1 in - sehr schematischer - Schnittansicht.

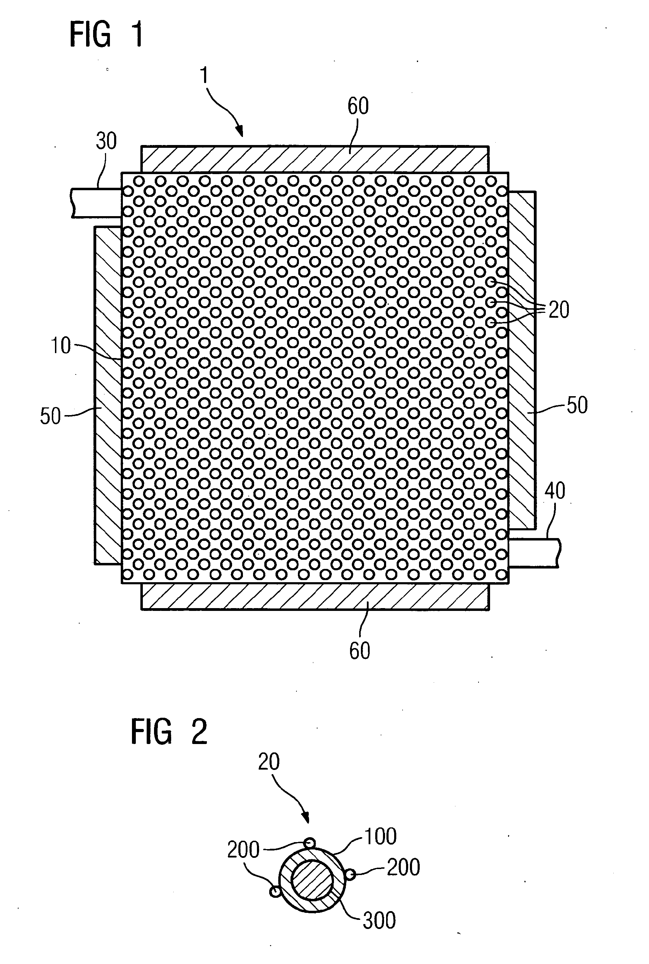

Fig. 1 zeigt ein Sterilisationssystem 1 in Form von Partikeln 20, angeordnet in einem Flussreaktor 10 gemäß einer ersten Ausführungsform der Erfindung in schematischer Schnittansicht. Die Partikel werden durch ein äußeres Magnetfeld in dem Flussreaktor auf gewünschte Weise fixiert. Hierzu sind in dieser Ausführungsform externe Magnete 50, 60 am Reaktor vorgesehen, die entweder als Permanentmagnete oder magnetisierbare elektrische Komponenten wie Spulen etc. ausgebildet sein können.

Das zu sterilisierende Fluid betritt den Reaktor beim Einlass 30 und verlässt diesen, nachdem es die Partikel 20 passiert hat, durch den Auslass 40.

Fig. 2 zeigt einen Partikel aus Fig.1 in - sehr schematischer - Schnittansicht. Dieser besteht aus einem Kern 300, welcher aus einem ferromagnetischen Material gemacht ist. Bevorzugt ist der Kern aus Eisen. Um den Kern herum befindet sich das Reduktionsmittel 100, welches in dieser Ausführungsform aus Silber besteht. Jedoch sind auch alle anderen zuvor genannten Reduktionsmittel denkbar. Neben dem Reduktionsmittel 100 sind mehrere Lokalelemente 200 angeordnet, die aus dem Oxidationsmittel bestehen. In dieser Ausführungsform ist das Oxidationsmittel Palladium, jedoch sind auch hier alle anderen zuvor genannten Oxidationsmittel denkbar.

Beim Passieren der Partikel 20 werden die Mikroorganismen in dem Fluid der Potentialdifferenz zwischen dem Reduktionsmittel 100 und dem Oxidationsmittel 200 ausgesetzt und das Fluid so sterilisiert.

## Patentansprüche

1. Sterilisationssystem für die Sterilisation und/oder Inaktivierung der Aktivität von Mikroorganismen in Flüssigkeiten und/oder Gasen, wobei das System Partikel umfasst, bei denen mindestens ein Oxidationsmittel und mindestens ein Reduktionsmittel so angeordnet sind, dass eine Potentialdifferenz von ≥200 bis ≤1000 mV erreicht wird,
**dadurch gekennzeichnet,**
**dass** die Partikel zusätzlich mindestens eine ferromagnetische Komponente enthalten und in einem Magnetfeld fixierbar sind.

2. Sterilisationssystem nach Anspruch 1,
**dadurch gekennzeichnet,**
**dass** die Partikel einen Partikeldurchmesser von ≥20 nm bis ≤10 µm aufweisen.

3. Sterilisationssystem nach einem der Ansprüche 1 bis 2,
**dadurch gekennzeichnet,**
**dass** das Oxidationsmittel und/oder das Reduktionsmittel eine Austauschstromdichte von ≥10⁻³ A/cm² bis ≤100 A/cm² aufweist.

4. Sterilisationssystem nach einem der Ansprüche 1 bis 3,
**dadurch gekennzeichnet,**
**dass** das Oxidationsmittel ausgewählt ist aus der Gruppe Ag, Au, Cu, Co, Ni oder Mischungen davon.

5. Sterilisationssystem nach einem der Ansprüche 1 bis 4,
**dadurch gekennzeichnet,**
**dass** das Reduktionsmittel ausgewählt ist aus der Gruppe Pd, Pt, Rh, Ir oder Mischungen davon.

6. Sterilisationssystem nach einem der Ansprüche 1 bis 5,
**dadurch gekennzeichnet,**
**dass** in den Partikel die ferromagnetische Komponente als Kern vorgesehen ist, welcher von dem Oxidazionsmittel und/oder dem Reduktionsmittel umgeben ist.

7. Verfahren zum Sterilisieren von Flüssigkeiten oder Gasen, enthaltend die Schritte:
- Bereitstellen eines Sterilisationssystems nach einem der Ansprüche 1 bis 6 in einem Reaktor
- Fixieren des Sterilisationssystems durch Anlegen eines magnetischen Feldes
- Durchleiten der zu sterilisierenden Flüssigkeit oder des Gases durch den Reaktor.

## Claims

1. Sterilization system for the sterilization and/or neutralization of the activity of microorganisms in liquids and/or gases, the system comprising particles in which at least one oxidizing agent and at least one reducing agent are arranged in such a way that a potential difference ranging from ≥ 200 to ≤ 1000 mV is achieved, **characterized in that** the particles additionally contain at least one ferromagnetic component and can be fixed in a magnetic field.

2. Sterilization system according to Claim 1, **characterized in that** the particles have a particle diameter ranging from ≥ 20 nm to ≤ 10 µm.

3. Sterilization system according to either of Claims 1 and 2, **characterized in that** the oxidizing agent and/or the reducing agent has an exchange current density ranging from ≥ 10⁻³ A/cm² to ≤ 100 A/cm².

4. Sterilization system according to one of Claims 1 to 3, **characterized in that** the oxidizing agent is selected from the group comprising Ag, Au, Cu, Co, Ni or mixtures thereof.

5. Sterilization system according to one of Claims 1 to 4, **characterized in that** the reducing agent is selected from the group comprising Pd, Pt, Rh, Ir or mixtures thereof.

6. Sterilization system according to one of Claims 1 to 5, **characterized in that** the ferromagnetic component is provided in the particles as a core which is surrounded by the oxidizing agent and/or the reducing agent.

7. Process for sterilizing liquids or gases, comprising the steps of
- providing a sterilization system according to one of Claims 1 to 6 in a reactor
- fixing the sterilization system by applying a magnetic field
- passing the liquid or gas that is to be sterilized through the reactor.

## Revendications

1. Système de stérilisation pour la stérilisation et/ou l'inactivation de l'activité de micro-organismes dans des liquides et/ou dans des gaz, dans lequel le système comprend des particules, dans lesquelles au moins un agent d'oxydation et au moins un agent de réduction sont mis, de manière à obtenir une différence de potentiel de ≥200 à ≤1000 mV,
**caractérisé**
**en ce que** les particules contiennent en plus au moins un constituant ferromagnétique et peuvent être immobilisées dans un champ magnétique.

2. Système de stérilisation suivant la revendication 1,
**caractérisé**
**en ce que** les particules ont un diamètre de particules de ≥20 nm à ≤10 µm.

3. Système de stérilisation suivant l'une des revendications 1 à 2,
**caractérisé**
**en ce que** l'agent d'oxydation et/ou l'agent de réduction ont une densité de courant d'échange de ≥10⁻³ A/cm² à ≤100 cm².

4. Système de stérilisation suivant l'une des revendications 1 à 3,
**caractérisé**
**en ce que** l'agent d'oxydation est choisi dans le groupe Ag, Au, Cu, Co, Ni ou leurs mélanges.

5. Système de stérilisation suivant l'une des revendications 1 à 4,
**caractérisé**
**en ce que** l'agent de réduction est choisi dans le groupe Pd, Pt, Rh, Ir ou leurs mélanges.

6. Système de stérilisation suivant l'une des revendications 1 à 5,
**caractérisé**
**en ce que** dans les particules le constituant ferromagnétique est prévu comme coeur, qui est entouré de l'agent d'oxydation et/ou l'agent de réduction.

7. Procédé de stérilisation de liquide ou de gaz, comportant les stades dans lesquels :
- on se procure un système de stérilisation suivant l'une des revendications 1 à 6 dans un réacteur
- on immobilise le système de stérilisation par application d'un champ magnétique
- on fait passer le liquide ou le gaz à stériliser dans le réacteur.
